# EUROPÄISCHE PATENTSCHRIFT

(11) **EP 0 624 364 B1**
(45) Veröffentlichungstag und Bekanntmachung des Hinweises auf die Patenterteilung: **24.07.1996**
(21) Anmeldenummer: 94105539.4
(22) Anmeldetag: 11.04.1994
(51) Int. Cl.: A61K 9/00

(54) **Brausesystem mit einem alkali- und/oder metallempfindlichen, pharmazeutischen Wirkstoff, und Verfahren zur Herstellung**
Effervescent system containing an alkalki- and/or metal sensitive pharmaceutical active, and method for producing it
Système effervescent contenant un principe actif sensible aux alcalis et/ou aux métaux, et procédé pour sa production

(30) Priorität: 15.04.1993 CH 1137/93
(43) Veröffentlichungstag der Anmeldung: 17.11.1994
(73) Patentinhaber: Gergely, Gerhard, Dr., A-1050 Wien (AT)
(72) Erfinder: Gergely, Gerhard, Dr., A-1050 Wien (AT); Gergely, Irmgard, A-1050 Wien (AT); Gergely, Thomas, Dr., A-1050 Wien (AT)
(74) Vertreter: Büchel, Kurt F., Dr.

(56) Entgegenhaltungen:
- EP-A- 0 181 564
- WO-A-93/00886
- US-A- 4 678 661

## Beschreibung

Die Erfindung betrifft eine pharmazeutische Zubereitung nach dem Oberbegriff des Anspruches 1, sowie ein Verfahren zu deren Herstellung.

Bei der derzeitigen Tendenz, immer mehr neue Wirkstoffe in Brausegranulate einzubauen, treten in zunehmendem Masse - bei der Verarbeitung besonders empfindlicher Wirkstoffe in Brausegranulate Instabilitäten auf. Solche Wirkstoffe kann man in Bezug zur Brause generell in zwei Gruppen einteilen: in Wirkstoffe, die extrem alkaliempfindlich sind, und in Wirkstoffe, die extrem säureempfindlich sind. Dazu kommt häufig eine hohe Empfindlichkeit gegen Metallionen, die dazu führen kann, dass eine gewisse Zersetzung oder ein Abbau schon bei Verwendung eines Drahtsiebes beginnt. Beispiele für alkaliempfindliche Wirkstoffe sind Acetylsalicylsäure, Pethidin, Chloramphenicol, Phenobarbital, Nicotinamid, Benzylpenicillin, sowie die ACE-Hemmer Enalapril, Perindopril-tertiäres Butylamin und Ramipril; Beispiele für Alkali- und Schwermetallionen-Empfindlichkeit sind Morphin, Acetylcystein, Ascorbinsäure, Thiamin (Vitamin B1), Riboflavin (Vitamin B2), Pyridoxin (Vitamin B6) und Cyanocobalamin (Vitamin B12).

Geht man bei diesen empfindlichen Wirkstoffen so vor, dass der Wirkstoff einer fertigen Brausemischung - gegebenenfalls mit zusätzlichen Füllstoffen - zugesetzt wird, um den Kontakt zur Brausemischung möglichst gering zu halten, so zeigt es sich häufig, dass es trotzdem zu einem Abbau des Wirkstoffes kommt. Bei solchen Systemen zeigen auch die Vitamine der B-Gruppe, wie Riboflavin, Pyridoxin und Thiamin, bereits einen Abbau bis zu 2%, nach 6 bis 12 Monaten einen solchen von bis zu 5%! Wird z. B. der Wirkstoff Captopril, der sehr alkali- und metallempfindlich ist, nach diesem Stand des Wissens einer Standard-Brausemischung zugefügt, so zeigt sich schon nach zwei Monaten ein Abbau des Captoprils durch Dimerisierung von ca. 6%. Schon beim Sieben durch ein gewöhnliches Drahtsieb kann es zu Dimerisierungen von 0,2% und mehr kommen. Andererseits hat das Brausegranulat auch noch genügend freie Alkalien, die in Kontakt mit dem Captopril kommen. Auch die Verankerung dieses Wirkstoffs auf einem Säurekristall mit Hilfe eines Bindemittels reicht für den Schutz nicht aus: nach 2 Monaten zeigt sich noch immer ein Abbau von ca. 4%. Ebenso bringt die Einbettung des Wirkstoffes in einen neutralen Füllstoff keine Lösung des Problems:

**Tabelle 1**

| Abbau nach 2 Mten: | |
|---|---|
| Captopril / Brausebasis | 6,06% |
| Captopril-Weinsäure-Phase / Brausebasis | 3,88% |
| Captopril-Mannit-Phase / Brausebasis | 3,18% |

Weiters war einerseits versucht worden, der Metallempfindlichkeit von Captopril durch Behandlung der Brausemischung mit einer Lösung von Äthylendiamintetraessigsäure (EDTA) entgegenzuwirken, und andererseits auch die Wirkstoffphase selbst mit EDTA zu behandeln. EDTA bildet mit Schwermetallen Komplexverbindungen, aus denen das Metall nicht mehr schädlich wirken kann. Diese Massnahmen und Kombinationen zeigen bereits eine Verbesserung des Abbauverhaltens, jedoch noch nicht in zufriedenstellendem Umfang:

**Tabelle 2**

| Abbau nach 2 Mten bei Raumtemperatur: | |
|---|---|
| Captopril + Weinsäure + EDTA / Brausebasis | 2,88% |
| Captopril + Weinsäure / Brausebasis + EDTA | 1,45% |
| Captopril + Weinsäure + EDTA / Brausebasis + EDTA | 0,80% |

Da diese Vorgangsweisen alleine nicht zielführend waren, mussten Strukturen gefunden werden, die dem Wirkstoff gegenüber Alkalien ausreichenden Schutz bieten. Nun ergab sich in überraschender Weise eine Lösung der geschilderten Probleme durch die im Kennzeichen des Anspruchs 1 geschilderten Massnahmen. Sie gelten für alle pharmazeutischen Wirkstoffe, die extrem alkaliempfindlich, und auch für solche, die alkali- und metallempfindlich sind. Vorteilhafte Weiterungen der Erfindung sind in den Kennzeichen der abhängigen Ansprüche beschrieben.

Das Prinzip beruht darauf, dass der Wirkstoff von wenigstens einer Säure - unter Ausschluss von Fumarsäure - oder einem Neutralstoff abgedeckt, vorzugsweise eingebettet ist. Nun wurde in der EP-A-181'564 bereits vorgeschlagen, Ibuprofen mit Fumarsäure zu ummanteln. Dort ist jedoch sowohl die Aufgabe der Erfindung als auch der sich durch die Lösung des Problems einstellende Effekt von der vorliegenden Anmeldung verschieden. Die schlechtlösliche Fumarsäure würde ihren Zweck zur Lösung der Aufgabe der vorliegenden Anmeldung nicht erfüllen können.

Vorteilhafterweise wird auch die Brausemischung so gestaltet, dass die Alkali- oder Erdalkalicarbonate oder -bicarbonate auf einen Träger aus kristalliner, organischer Säure mit pulverisierter Zitronensäure oder in Mischung mit pulverisierter Säure aufgetragen werden, worauf diese Schicht noch mit Hilfe einer weiteren pulverisierten, essbaren organischen Säure - mittels einer Säurelösung als Bindemittel - abgedeckt wird, so dass die Kontaktzone zwischen Wirkstoff und Brausegranulat, bzw. die Oberfläche der Teilchen des Brausesystems einen pH-Wert von höchstens 4,5 aufweist. Dieser kann bestimmt werden, indem einzelne Granulatkörner auf einem befeuchteten pH-Papier abgerollt werden, worauf man an Hand der Farbentwicklung den pH-Wert feststellt.

Das gesteckte Ziel kann man auch erreichen, indem man die Carbonate und/oder Bicarbonate gemeinsam mit pulverisierter Zitronensäure granuliert, sodass sie eingebettet sind, und dieses so hergestellte Granulat mittels einer Säurelösung noch mit einer Schicht von einer pulverisierten, essbaren, organischen Säure abdeckt.

Je nachdem, wie gut die Einbettung ist, kann gegebenenfalls auf die zusätzliche Abdeckung auch verzichtet werden. Werden Vitamine der B-Gruppe, z.B. Pyridoxin, mit solcherart hergestellten Brausegranulaten gemischt und zu Tabletten verpresst, so zeigen sich bei Lagerung bereits eine verbesserte Stabilität und ein um etwa die Hälfte verringerter Abbau.

Andererseits zeigt sich beispielsweise, dass mit ein und derselben Captopril-Phase das Produkt nur dann eine wesentlich verbesserte Stabilität aufweist, wenn die Oberfläche des Brause-Grundgranulates mit einer Säureschicht abgedeckt wurde.

Während eine Brausebasis nach Beispiel 2 aus 50 % Zitronensäure-Träger, 30 % Natriumcarbonat-EDTA/Zitronensäurelösung und nur 7 % Abdeckung mit Zitronensäurepulver nach 3 Monaten einen Abbau von immerhin noch 0,70% zeigt, zeigt eine Brausebasis gleicher Zusammensetzung mit einer Zitronensäureabdeckung von 20 % nach 3 Monaten bei Raumtemperatur einen Abbau von nur noch 0,18%.

Man erreicht das dadurch, dass man (vorzugsweise im Vakuum) Zitronensäure-Trägerkristalle mit der erforderlichen Menge Alkalibicarbonat und pulverisierter organischer Säure, durch Zugabe von Wasser, Äthanol, oder einer Mischung von beiden anreagieren lässt, das Produkt trocknet und hernach mit Zitronensäure-Lösung (äthanolisch, oder wässerig, oder Mischung) benetzt und mit pulversierter Zitronensäure, Fumarsäure, Adipinsäure oder Äpfelsäure - allseitig geschlossen - abdeckt. Die dazu notwendigen Lösungen können bereits EDTA enthalten, bzw. kann EDTA in separaten Lösungen aufgetragen werden.

Obwohl eine Wirkstoffphase mit einem ausreichenden Gehalt an EDTA in der Abdeckung - z.B. 0.05 bis 0.5 Gewichtsteile bei Aufbringen in wässeriger Lösung oder Suspension bzw. bis zu 2 Gewichtsteile bei Aufbringen des trockenen Pulvers, bezogen auf 100 Gewichtsteile der gesamten Wirkstoffphase, insbesondere 0.4 bis 2 Gewichtsteile auf 100 Gewichtsteile Wirkstoff - schon eine gute Stabilität in Mischung mit einem erfindungsgemäss abgedeckten Brausegranulat zeigt, hat sich in vielen Fällen doch auch ein Gehalt an EDTA in der Abdeckung des Brausegranulats als zweckmässig erwiesen, und zwar 0.01 bis 0.5 Gewichtsteile bei Aufbringen in wässeriger Lösung oder Suspension bzw. 0.1 bis 2 Gewichtsteile bei Aufbringen des trockenen Pulvers, bezogen auf 100 Gewichtsteile des Brausegranulats. Das Aufbringen in wässeriger Lösung ist aber bevorzugt. Die Gesamtmenge an EDTA in einer Brausetablette liegt insbesondere im Bereich von 0.1 bis 3 mg pro Tablette.

Für die Herstellung des Brausegranulates gibt es - wie oben bereits erwähnt - zwei Verfahren, um die Alkalien einzubetten:

### Beispiel 1:

3000 Teile einer kristallisierten Zitronensäure, 1000 Teile pulverisierter Zitronensäure, 2000 Teile Natriumbicarbonat und 300 Teile Natriumcarbonat werden mit Äthanol so granuliert, dass die Alkalien auf der kristallisierten und in der pulverisierten Zitronensäure eingebettet sind; anschliessend wird das Produkt getrocknet, entweder bei 70°C oder mittels Vakuum bei 60°C auf 15 mbar. Das resultierende Granulat wird dann mit einer Lösung von 400 Teilen Zitronensäure und 1 bis 5 Teilen EDTA in 200 ml Wasser-Äthanol 1:1 benetzt, mit 1000 Teilen pulverisierter Zitronensäure abgedeckt und anschliessend getrocknet.

### Beispiel 2:

Nimmt man pulverisierte Zitronensäure zur Einbettung von Alkalien oder Erdalkalien, so kann man folgendermassen vorgehen: 4000 Teile pulverisierte Zitronensäure werden mit 1700 Teilen pulverisiertem Natriumbicarbonat und 500 Teilen Natriumcarbonat mit einer Lösung von 200 g Zitronensäure in 130 ml Äthanol so behandelt, dass von dieser Lösung zwei Mal 80 ml aufgebracht werden; dazwischen wird getrocknet.

Vor dem Trocknen der zweiten Lösung wird nochmals eine EDTA-Lösung aufgebracht und anschliessend mit 1000 Teilen Zitronensäure pulvis abgedeckt. Das Granulat wird sodann bei Temperaturen von 80°C oder im Vakuum unter 10 mbar getrocknet.

### Beispiel 3:

Es kann auch Weinsäure als Träger Verwendung finden; das Granulat wird dann ganz entsprechend Beispiel 1 hergestellt.

### Beispiel 4:

Es können auch 500 Teile Fumarsäure zum Abdecken Verwendung finden; das Granulat wird dann ganz entsprechend Beispiel 2 hergestellt.

### Beispiel 5:

Man arbeitet entsprechend dem Beispiel 2, bettet jedoch an Stelle von Natriumbicarbonat und Natriumcarbonat Calciumcarbonat in die Säure ein.

Der zweite notwendige Schritt besteht darin, den alkaliempfindlichen Wirkstoff selbst auf eine organische Säure aufzubringen und dort zu verankern, oder in eine Säure einzubetten. Von diesen Grundprinzipien ausgehend, zeigt sich, dass es nicht genügt, den Wirkstoff auf der Zitronensäure zu verankern, sondern der Wirkstoff muss zusätzlich mit einer Säure und/oder einem Hydrokolloid und/oder mit einem höherwertigen Alkohol, wie z.B. Mannitol oder Sorbitol, abgedeckt werden, sodass er gegen den Einfluss von Alkalien geschützt ist. Dazu kommen Verbindungen wie Maltodextrin, Guargum, Gelatine, oder auch Gummi arabicum in Frage. Auch hier wird bei den Lösungen, die dazu notwendig sind, vorteilhafterweise EDTA zugefügt. Bei der Kombination verschiedener Wirkstoff-Phasen mit der gleichen Brausebasis zeigten sich die entsprechenden Unterschiede hinsichtlich Stabilität:

**Tabelle 3**

| Abbau nach 3 mon bei Raumtemperatur: | |
|---|---|
| Brausebasis nach Beispiel 2 - Captoprilphase A1 | 0,54 |
| Brausebasis nach Beispiel 2 - Captoprilphase A2 | 0,78 |
| Brausebasis nach Beispiel 2 - Captoprilphase A10 | 0,28 |
| Brausebasis nach Beispiel 2 - Captoprilphase A12 | 0,28 |
| Erläuterung der Wirkstoff-Phasen: A1: Captopril wird mit einer PVP-Lösung, in der EDTA gelöst ist, an der Oberfläche von Weinsäure verankert. A2: Captopril wird mit einer PVP-Lösung, in der EDTA gelöst ist, auf Zitronensäure verankert. A10: Captopril wird mit einer wässrigen EDTA-Lösung auf der Weinsäure verankert und anschliessend mit Maltodextrin und Fumarsäure abgedeckt. A12: Captopril wird mit einer wässrigen Vitamin C- und EDTA-Lösung auf der Weinsäure verankert und anschliessend mit Fumarsäure und Maltodextrin abgedeckt. | |

In A10 und A12 wirkt nicht die Fumarsäure erfindungsgemäss, sondern der daneben aufgebrachte Neutralstoff Maltodextrin bzw. Mannit.

Sowohl die Weinsäure-Oberfläche als auch - nur geringfügig, aber immerhin - die Captopril-Oberfläche werden nämlich angelöst, sodass sie klebrig werden und das Maltodextrin sowie die Fumarsäure darauf haften bleiben.

Es kann auch Vitamin C oder Tocopherolacetat als Radikalfänger in der Wirkstoff-Phase eine Stabilitätsverbeserung bringen. Dabei zeigt sich, dass auch die optimale Einbettung des Wirkstoffes in die Säurephase für die Stabilität eine wesentliche Rolle spielt.

Die folgenden Beispiele zeigen das Prinzip eines optimalen Schutzes des Wirkstoffes durch Einbettung:

### Beispiel 6:

25 Teile Captopril werden mit 90 Teilen gemahlenem Vitamin C vermischt und mit Hilfe einer Lösung von 10 Teilen Zitronensäure und 0,2 Teilen EDTA in 2 Teilen Alkohol und 4 Teilen Wasser granuliert. Darauf werden 25 Teile Maltodextrin und 10 Teile Fumarsäure aufgebracht und bei 60°C - vorzugsweise mittels Vakuum - getrocknet.

### Beispiel 7:

25 Teile Captopril werden mit 100 Teilen Weinsäure vermischt und mit einer Lösung von 10 Teilen Maltodextrin in 5 Teilen Wasser und 0,2 Teilen EDTA benetzt. Sodann wird mit 50 Teilen Mannit und 20 Teilen Fumarsäure abgedeckt und getrocknet.

### Beispiel 8:

25 Teile Captopril werden mit 100 Teilen Zitronensäure pulvis vermischt und mit einer Lösung aus 0,2 Teilen EDTA und 1 Teil Wasser benetzt; anschliessend wird mit einer Lösung aus 1 Teil PVP in 3 Teilen Äthanol granuliert und mit 10 Teilen pulverisiertem Vitamin C abgedeckt.

### Beispiel 9:

10 Teile Riboflavin werden mit 5 Teilen pulverisierter Äpfelsäure gemischt, mit 1 Teil PVP in Äthanol-Lösung granuliert und anschliessend mit 10 Teilen pulverisiertem Sorbitol und 10 Teilen Zitronensäure abgedeckt.

### Beispiel 10:

10 Teile Pyridoxin-Hydrochlorid werden mit 80 Teilen Zitronensäure vermischt und mit einer Lösung von 1 Teil Ascorbinsäure und 0,2 Teilen EDTA in 3 Teilen Wasser granuliert; das Granulat wird anschliessend mit 10 Teilen pulverisierter Zitronensäure abgedeckt.

Kombinationen dieser Wirkstoff-Phasen mit den angeführten Brausebasen zeigen nach einer Lagerung von 2 Monaten bei Raumtemperatur einen Abbau von nur 0,03 - 0,2 %.

Die Erfindung gilt über die angeführten Beispiele hinaus für alle alkali- und/oder metallempfindlichen Wirkstoffe; auch können andere essbare, organische Säuren zum Einsatz gelangen.

## Patentansprüche

1. Pharmazeutische Zubereitung in Form einer Mischung von wenigstens einem alkali- und/oder metallempfindlichen Wirkstoff mit einem Brausesystem aus wenigstens einem Alkali- oder Erdalkalicarbonat oder -bicarbonat und wenigstens einer festen, essbaren, organischen Säure, dadurch gekennzeichnet, dass der Wirkstoff von wenigstens einer der folgenden Verbindungen: eine feste, essbare, organische Säure - unter Ausschluss von Fumarsäure -, wie z.B. Zitronensäure, Weinsäure, Äpfelsäure, Ascorbinsäure, Adipinsäure; ein höherwertiger Alkohol, wie z.B. Mannit, Sorbit, Xylit; ein Hydrokolloid, wie z.B. Maltodextrin, Guargum, Gelatine oder Gummi arabicum; Lösung eines höhermolekularen Polyvinylpyrrolidons abgedeckt, vorzugsweise in eine solche Verbindung eingebettet ist, wobei die Zubereitung gegebenenfalls zu Tabletten verpresst ist.

2. Zubereitung nach Anspruch 1, dadurch gekennzeichnet, dass die karbonatischen und wenigstens ein Teil der sauren Bestandteile des Brausesystems auf Trägerkristallen wenigstens einer festen, essbaren, organischen Säure - vorzugsweise in Mischung mit oder eingebettet in dieselbe oder eine andere essbare, organische Säure - aufgebracht und gegebenenfalls auch mit derselben oder einer anderen festen, essbaren, organischen Säure abgedeckt sind.

3. Zubereitung nach Anspruch 1 oder 2, dadurch gekennzeichnet, dass die Wirkstoffteilchen auf Trägerkristallen wenigstens einer festen, essbaren, organischen Säure aufgebracht und entweder eingebettet in wenigstens eine oder abgedeckt mit wenigstens einer der genannten Verbindungen sind.

4. Zubereitung nach Anspruch 1, dadurch gekennzeichnet, dass die carbonatischen und wenigstens ein Teil der sauren Bestandteile des Brausesystems granuliert und vorzugsweise mit derselben oder einer anderen festen, essbaren, organischen Säure abgedeckt sind.

5. Zubereitung nach Anspruch 1, dadurch gekennzeichnet, dass die Wirkstoffteilchen in Mischung mit wenigstens einer der genannten Verbindungen vorliegen.

6. Zubereitung nach einem der vorhergehenden Ansprüche, dadurch gekennzeichnet, dass die für die Einbettung bzw. Abdeckung vorgesehene Säure Äthylendiamintetraessigsäure enthält, und zwar bevorzugt 0,01 bis 2 Gewichtsteile für die Abdeckung von 100 Gewichtsteilen Brausegranulat, bzw. bevorzugt 0,05 bis 2 Gewichtsteile für die Abdeckung von 100 Gewichtsteilen Wirkstoffphase.

7. Zubereitung nach einem der vorhergehenden Ansprüche, dadurch gekennzeichnet, dass der Wirkstoff ein ACE-Hemmer, insbesondere Captopril ist.

8. Zubereitung nach einem der vorhergehenden Ansprüche, dadurch gekennzeichnet, dass die Aussenschicht des Brausesystems einen pH-Wert von höchstens 4,5 aufweist.

9. Verfahren zur Herstellung einer Zubereitung nach einem der vorhergehenden Ansprüche, dadurch gekennzeichnet, dass getrennt voneinander je eine Brausephase und eine Wirkstoffphase hergestellt und anschliessend gemischt, sowie gegebenenfalls zu Tabletten verpresst werden, wobei die Brausephase durch Einbetten pulverisierter Alkali- und/oder Erdalkalicarbonate bzw. -bicarbonate, die Wirkstoffphase durch Einbetten pulverisierter Wirkstoffteilchen, jeweils in Granulatkörner aus einer essbaren, organischen Säure, gebildet werden.

10. Verfahren nach Anspruch 9, dadurch gekennzeichnet, dass die Granulatkörner der Brausephase und/oder der Wirkstoffphase von einer Schicht aus wenigstens einer der folgenden Verbindungen: eine essbare, organische Säure, wie z.B. Zitronensäure, Weinsäure, Äpfelsäure, Ascorbinsäure, Adipinsäure; ein höherwertiger Alkohol, wie z.B. Mannit, Sorbit, Xylit; ein Hydrokolloid, wie z.B. Maltodextrin, Guargum, Gelatine oder Gummi arabicum; Lösung eines höhermolekularen Polyvinylpyrrolidons - gegebenenfalls in Gegenwart von Äthylendiamintetraessigsäure - vorzugsweise in einer Vakuum-Mischtrommel, abgedeckt werden.

11. Verfahren nach Anspruch 10, dadurch gekennzeichnet, dass in die Schichte etwa 1 bis etwa 5 mg pro Tablettenmenge an Äthylendiamintetraessigsäure eingebaut wird.

## Claims

1. Pharmaceutical formulation in the form of a mixture of at least one alkali- and/or metal-sensitive active ingredient with an effervescent system comprising at least one alkali metal carbonate or alkaline earth metal carbonate or alkali metal bicarbonate or alkaline earth metal bicarbonate and at least one solid, edible, organic acid, characterized in that the active ingredient is covered by at least one of the following compounds: a solid, edible, organic acid - excluding fumaric acid - such as citric acid, tartaric acid, malic acid, ascorbic acid or adipic acid; a polyhydric alcohol, such as, for example, mannitol, sorbitol, or xylitol; a hydrocolloid, such as, for example, maltodextrin, guar gum, gelatine or gum arabic; a solution of a relatively high molecular weight polyvinylpyrrolidone, preferably embedded in such a compound, the formulation optionally being compressed to give tablets.

2. Formulation according to Claim 1, characterized in that the carbonate components and at least a part of the acidic components of the effervescent system are applied to carrier crystals of at least one solid, edible, organic acid - preferably as a mixture with or embedded in the same or another edible, organic acid - and are optionally also covered with the same or another solid, edible, organic acid.

3. Formulation according to Claim 1 or 2, characterized in that the active ingredient particles are applied to carrier crystals of at least one Solid, edible, organic acid and either embedded in at least one, or covered with at least one, of the stated compounds.

4. Formulation according to Claim 1, characterized in that the carbonate components and at least a part of the acidic components of the effervescent system are granulated and are preferably covered with the same or another solid, edible, organic acid.

5. Formulation according to Claim 1, characterized in that the active ingredient particles are present as a mixture with at least one of the stated compounds.

6. Formulation according to any of the preceding Claims, characterized in that the acid provided for the embedding or covering contains ethylenediaminetetraacetic acid, preferably 0.01 to 2 parts by weight for covering 100 parts by weight of effervescent granules, and preferably 0.05 to 2 parts by weight for covering 100 parts by weight of active ingredient phase.

7. Formulation according to any of the preceding Claims, characterized in that the active ingredient is an ACE inhibitor, in particular captopril.

8. Formulation according to any of the preceding Claims, characterized in that the outer coat of the effervescent system has a pH of not more than 4.5.

9. Process for the preparation of a formulation according to any of the preceding Claims, characterized in that an effervescent phase and an active ingredient phase are prepared separately from one another and then mixed and optionally compressed to give tablets, the effervescent phase being formed by embedding powdered alkali metal and/or alkaline earth metal carbonates or bicarbonates, and the active ingredient phase by embedding powdered active ingredient particles, in each case in granular particles comprising an edible, organic acid.

10. Process according to Claim 9, characterized in that the granular particles of the effervescent phase and/or of the active ingredient phase are covered by a coat of at least one of the following compounds: an edible, organic acid, such as, for example, citric acid, tartaric acid, malic acid, ascorbic acid or adipic acid; a polyhydric alcohol, such as, for example, mannitol, sorbitol or xylitol; a hydrocolloid, such as, for example, maltodextrin, guar gum, gelatine or gum arabic; a solution of a relatively high molecular weight polyvinylpyrrolidone - optionally in the presence of ethylenediaminetetraacetic acid - preferably in a vacuum mixing drum.

11. Process according to Claim 10, characterized in that about 1 to about 5 mg of ethylenediaminetetraacetic acid are embedded in the coat per tablet weight.

## Revendications

1. Préparation pharmaceutique se présentant sous la forme d'un mélange d'au moins une substance active à sensibilité aux alcalis et/ou aux métaux, ayant un système effervescent constitué d'au moins un carbonate ou un bicarbonate alcalin ou alcalino-terreux et d'au moins un acide résistant, comestible, organique, caractérisée en ce que la substance active est recouverte par au moins l'une des combinaisons ci-après : un acide résistant, comestible, organique - à l'exclusion de l'acide fumarique - tel que l'acide citrique, l'acide tartrique, l'acide malique, l'acide ascorbique, l'acide adipique; un alcool supérieur, tel que par exemple la mannite, la sorbite, la xylite; un hydrocolloïde, tel que par exemple la maltodextrine, le guargum, la gélatine ou la gomme arabique; une solution d'un polyvinylpyrrolidon à poids moléculaire élevé, de préférence intégrée dans une telle combinaison, la préparation étant le cas échéant pressée pour donner des comprimés.

2. Préparation selon la revendication 1, caractérisée en ce que les éléments carbonatés et au moins une partie des composants acides du système effervescent sont appliqués sur des cristaux support d'au moins un acide résistant, comestible, organique - de préférence un mélange comportant, ou étant noyé dans, le même ou un autre acide comestible organique - et le cas échéant également recouvert du même acide ou d'un autre acide résistant, comestible, organique.

3. Préparation selon la revendication 1 ou 2, caractérisée en ce que les particules de substance active sont appliquées sur des cristaux support d'au moins un acide résistant, comestible, organique et sont soit intégrées dans au moins une, soit recouverts par, au moins l'une des combinaisons souhaitées.

4. Préparation selon la revendication 1, caractérisée en ce que les éléments carbonatés et au moins une partie des composants acides du système effervescent sont granulés et de préférence recouverts du même ou d'un autre acide résistant, comestible, organique.

5. Préparation selon la revendication 1, caractérisée en ce que les particules de substance active se présentent sous forme d'un mélange avec au moins l'une des combinaisons citées.

6. Préparation selon l'une des revendications précédentes, caractérisée en ce que l'acide prévu pour l'intégration ou le recouvrement contient de l'acide éthylendiamintétracétique, et notamment de préférence de 0,01 à 2 parties en poids pour le recouvrement de 100 parties en poids de granulés effervescents, respectivement de préférence de 0,05 à 2 parties en poids pour le recouvrement de 100 parties en poids de substance active.

7. Préparation selon l'une des revendications précédentes, caractérisée en ce que la substance active est un ACE-Hemmer, en particulier du captopril.

8. Préparation selon l'une des revendications précédentes, caractérisée en ce que la couche extérieure du système effervescent présente un pH d'une valeur maximale de 4,5.

9. Procédé de fabrication d'une préparation selon l'une des revendications précédentes, caractérisé en ce que l'on fabrique séparément les uns des autres respectivement une phase effervescente et une phase de substance active et l'on mélange ensuite, ainsi que, le cas échéant, on presse pour donner des comprimés, la phase effervescente étant constituée par intégration de carbonate ou de bicarbonate alcalin et/ou alcalino-terreux pulvérisé, la phase de substance active étant constituée par intégration de particules de substance active pulvérisées, chaque fois dans des grains de granulé constitués d'un acide comestible organique.

10. Procédé selon la revendication 9, caractérisé en ce que les grains de granulé de la phase effervescente et/ou de la phase à substance active sont recouverts par une couche d'au moins l'une des combinaisons ci-après : un acide comestible organique, tel que par exemple l'acide citrique, l'acide tartrique, l'acide malique, l'acide ascorbique, l'acide adipique; un alcool supérieur, tel que par exemple la mannite, la sorbite, la xylite; un hydrocolloïde, tel que par exemple la maltodextrine, le guargum, la gélatine ou la gomme arabique: une solution d'un polyvinylpyrrolidon à poids moléculaire élevé - le cas échéant en présence d'acide éthylendiamintétracétique - de préférence dans un tambour de mélange travaillant sous vide.

11. Procédé selon la revendication 10, caractérisé en ce que l'on intègre dans la couche environ 1 à environ 5 mg par quantité de tablette d'acide éthylendiamintétracétique.
